# EUROPEAN PATENT APPLICATION

(11) **EP 3 680 911 A1**
(43) Date of publication of application: **15.07.2020**
(21) Application number: 19151136.9
(22) Date of filing: 10.01.2019
(51) Int. Cl.: G16H 30/40, G16H 30/20, G16H 40/40

(54) **TECHNIQUE FOR CONFIGURING A MEDICAL IMAGING DEVICE**

(71) Applicant: Medneo GmbH, 10117 Berlin (DE)
(72) Inventor: HARTKENS, Thomas, 13086 Berlin (DE); ISSING, Matthias, 12307 Berlin (DE); MEZEY, David, 13357 Berlin (DE); VOGT, Johannes, 10827 Berlin (DE); JUNEJA, Medha, 10781 Berlin (DE)
(74) Representative: Laqua, Bernd Christian Kurt

(57) **Abstract**

A technique for configuring a medical imaging device by a set of acquisition parameters for acquiring a medical image is disclosed. A method implementation of the technique comprises selecting (S102) a reference image from a plurality of reference images, each of the plurality of reference images being stored in association with a set of acquisition parameters which has been used to acquire the respective reference image, and configuring (S104) the medical imaging device by the set of acquisition parameters stored in association with the selected reference image for acquiring the medical image.

## Description

### Technical Field

The present disclosure generally relates to the field of medical imaging systems. In particular, a method and system for configuring a medical imaging device by a set of acquisition parameters for acquiring a medical image is presented.

### Background

Quality control of medical images, such as magnetic resonance (MR) or computer tomography (CT) images, has been an intense field of research in the last decades. Generally, it is important to exclude problematic image acquisitions and avoid bias in the subsequent reading of images by physicians. Since visual inspection to detect images of bad quality is not always practical in the daily routine of radiological centers, quantitative image quality measurement techniques have been introduced which allow automatically verifying image quality after acquisition. However, quantitative measurements may not capture all aspects of image quality and, thus, quality-controlled acquisition based on purely quantitative measurements may not differentiate images sufficiently.

Image quality can be influenced by many variables, such as the acquisition parameters of the imaging device, the patient positioning in the medical imaging device (e.g., a scanner), the training of the staff, and the standard operating procedures (SOPs) to be applied for image acquisitions. In particular, an imaging device typically holds several hundreds or even more than a thousand different acquisition parameter protocols, which may be set depending on the specific needs of an examination. Radiological centers continuously modify those variables aiming to enhance the resulting image quality. However, especially in case of geographically distributed imaging devices (e.g., located in different buildings or different cities), the control of image quality may become challenging because all sites must generally use the same and up-to-date variables to achieve comparable image quality across all locations and to meet the expectations of the radiologists.

In case of distributed radiological centers, the following problems may particularly arise when it comes to the control of image quality. In conventional systems, on-site technicians must manually select the appropriate acquisition parameters from a list of generic acquisition protocols locally installed on the medical imaging device (also called "modality"). Due to the manual selection, a technician may choose - either by accident or by lack of experience - a wrong acquisition protocol for a particular patient. As the acquisition protocols are stored locally at each individual imaging device, inconsistencies in the parameters may occur across sites and the resulting imaging quality may hence vary between different radiological centers. If the acquisition parameters of an acquisition protocol stored at one site are not up-to-date, an obsolete protocol may be applied by the technician and, if SOPs are not properly synchronized with the selected acquisition parameters, motion artifacts may be introduced or wrong fields of view may be acquired, e.g., when the patient is not properly positioned in the scanner in accordance with an SOP. For a reliable diagnosis, the radiologist (e.g., working from a remote location) may need adapted acquisition parameters for a specific patient. As technicians typically adjust those parameters by themselves or after verbal communication with the radiologist, inappropriate adjustments may occur. Also, technicians at the modality may not recognize problems with the image quality and automatic quantitative quality measurements may not determine whether sufficient image quality is given with satisfying certainty, as described above. As a result, images of inferior quality may be sent to the radiologists and erroneous conclusions by the radiologists may be the consequence. Also, a re-acquisition of the image may not be possible since the patient may have left the imaging device by the time the radiologist reviews an image of inferior quality.

### Summary

Accordingly, there is a need for a technique for use in a medical imaging system that avoids one or more of the problems discussed above, or other problems.

According to a first aspect, a method for configuring a medical imaging device by a set of acquisition parameters for acquiring a medical image is provided. The method comprises selecting a reference image from a plurality of reference images, each of the plurality of reference images being stored in association with a set of acquisition parameters which has been used to acquire the respective reference image, and configuring the medical imaging device by the set of acquisition parameters stored in association with the selected reference image for acquiring the medical image.

Rather than configuring the medical imaging device by selecting a generic acquisition protocol stored locally at the medical imaging device, as described above, the medical imaging device may thus be configured by selecting a reference image. As the reference image is stored in association with the set of acquisition parameters which has been used to acquire the reference image, the medical imaging device may be configured by the same configuration that was used to acquire the reference image, in the expectation that the resulting imaging quality for the acquired image may be comparable to that of the reference image. The reference image may be selected from a plurality of reference images (e.g., from a pool of reference images stored in a repository) by a user, such as a physician (e.g., a radiologist) or other medical personnel. The selection of the reference image may be performed using a composition component of the medical imaging system which may be configured to receive a corresponding selection of the user (e.g., via a user interface). The composition component may be part of the medical imaging device itself or may be provided as a separate component in the medical imaging system. Once selected, the set of acquisition parameters stored in association with the selected reference image may be applied by a configuration component to the medical imaging device to effectively configure the medical imaging device. If the composition component is a separate component, the selected reference image may be transferred to the medical imaging device, e.g., using the digital imaging and communications in medicine (DICOM) standard, and, once transferred, the set of acquisition parameters stored in association with the selected reference image (e.g., stored together with the reference image in a DICOM file) may be applied to the medical imaging device.

Each of the plurality of reference images may represent a sample image usable as template for future image acquisitions. Each of the plurality of reference images may previously be selected as being representative of a desired level of image quality for a particular type of medical image. In other words, the plurality of reference images may correspond to images which are selected to be of "good" quality for particular types of medical images. The particular type of a medical image may relate to a particular body region or a particular anatomical part, for example. The plurality of reference images may be selected (or "exported") from a picture archiving and communication system (PACS), which may store a collection of previously acquired medical images. The selection of the plurality of reference images from the PACS may be performed by users with medical expertise, such as physicians (e.g., radiologists) or other medical personnel, for example. In another variant, the plurality of reference images may be determined automatically based on statistical methods or machine learning techniques and may be based on feedback on the image quality levels of the reference images previously collected from physicians, for example.

The medical imaging device may be a device configured to acquire structural or functional images of a body, including modalities using MR, CT, radiography, ultrasound, nuclear medicine and visible light, for example. The medical images acquired by the medical imaging device may correspond to MR images, CT images, X-ray images, etc. accordingly. Acquisition parameters may generally be used to configure the medical imaging device for a particular acquisition to be performed. As a mere example, typical acquisition parameters applied to an MR scanner may comprise definitions of a repetition time, an echo time, a number of signal averages, a slice thickness, an acquisition plane and a field of view, for example.

The medical imaging device may be part of a medical imaging system and may be installed at a particular site (e.g., at a radiological center). In one variant, the medical imaging device may be one of a plurality of medical imaging devices distributed across a plurality of sites (e.g., plural radiological centers in different buildings or different cities), wherein the plurality of reference images may be stored in a central repository configured to distribute the plurality of reference images to the plurality of medical imaging devices. In the central repository, the plurality of reference images may be centrally version controlled. In each of the plurality of sites, a local subset of the central repository may be maintained, wherein each local subset (or "local repository instance") may be synchronized with the central repository. The required data transfer may be realized over corresponding network connections, such as over the Internet, for example. A local repository instance may form the basis from which the reference image is selected by means of the composition component, as described above.

In the central repository, the plurality of reference images may be categorized by at least one of medical imaging device types, standard image sets and/or physician specific image sets, body regions, and anatomical parts. The central repository may thus be organized in categories to provide support for different types of imaging devices and to facilitate the retrieval of reference images. Among the above-mentioned categories, the category "medical imaging device type" may indicate for a reference image a type of medical imaging device on which the reference image has been acquired, the category "standard image set" may indicate for a reference image that the reference image is representative of a generally recommended set of acquisition parameters (e.g., independently from personal preferences of a particular physician), the category "physician specific image set" may indicate for a reference image that the reference image is representative of a set of acquisition parameters preferred by a particular physician, the category "body region" may indicate for a reference image a body region for which the reference image is representative (e.g., head, lower extremities, etc.), and the category "anatomical part" may indicate for a reference image an actual anatomical part for which the reference image is representative (e.g., ankle, foot, knee, etc.). Local repository instances may be organized in the same manner.

In the central repository, each of the plurality of reference images may further be stored in association with an SOP to be adhered to when acquiring the medical image using the set of acquisition parameters stored in association with the respective reference image. The SOP may contain instructions relating to variables not covered by the set of acquisition parameters stored in association with the respective reference image, such as instructions for the positioning of a patient (e.g., the positioning and orientation of a patient's body part in a scanner). While the set of acquisition parameters stored in association with the selected reference image may be applied to the medical imaging device directly, as described above, it will be understood that the set of acquisition parameters stored in association with the selected reference image may also be adjusted prior to configuring the medical imaging device. Such adjustments may be carried out using the composition component mentioned above, for example.

In order to obtain a whole series of images for a complete examination of a patient, sequences of reference images may be defined. In the central repository, sequences of images may thus be stored, wherein the medical imaging device may be configurable by each of the sequences to acquire a respective series of medical images in accordance with the sets of acquisition parameters stored in association with the reference images of the respective sequence. Instead of selecting a single reference image and configuring the medical imaging device based on the selected single reference image, as described above, the method may thus also comprise selecting a sequence of reference images from the sequences of reference images, and configuring the medical imaging device to acquire a series of medical images in accordance with the sets of acquisition parameters stored in association with the reference images of the selected sequence. In order to avoid duplicate storage of reference images in the central repository, reference images of the sequences may be stored by reference (or "be linked") to corresponding reference images of the plurality of reference images stored in the central repository.

A maintenance component configured to maintain consistency for these links may be provided. The maintenance component may be configured to update corresponding links if a reference image is removed to prevent a degenerated repository. The maintenance component may also be configured to identify reference images with similar acquisition parameters and, if reference images with sufficiently similar acquisition parameters are identified, to remove such reference images from the repository. While the selected sequence may be applied to the medical imaging device directly, as described above, it will be understood that a sequence selected from the sequences of reference images may be adjusted prior to configuring the medical imaging device with the selected sequence. For example, one or more reference images may be added or removed from the sequence in order to create a patient specific examination specifically adapted for a particular patient. Such adjustments may be carried out using the composition component mentioned above.

Once the medical imaging device is configured using the selected reference image, the medical image may effectively be acquired and, upon completion of the acquisition, an assessment of the obtained image quality may be carried out. In one variant, the method may thus comprise acquiring the medical image by the medical imaging device, and determining a level of image quality for the acquired medical image using an assessment component configured to assess image quality levels based on feedback of physicians collected for previously acquired medical images. The assessment of the image quality level carried out by the assessment component may (but does not necessarily have to) include applying quantitative image quality measurements, such as the conventional quantitative quality measurement techniques mentioned above. Rather than purely relying on quantitative image quality measurements for the determination of the image quality level of the acquired medical image, however, the assessment component may carry out the assessment based on feedback of physicians (e.g., radiologists) collected for previously acquired medical images. Such assessment may include calculating predictions of satisfaction levels of physicians using artificial intelligence-based techniques like deep learning, for example. In one variant, the assessment component may thus comprise a machine learning module trained to predict a satisfaction level of a physician or a group of physicians based on the feedback of physicians collected for previously acquired medical images. If the machine learning module is trained to predict the satisfaction level for a group of physicians, the satisfaction level may be representative of a general image quality (e.g., independently from personal preferences of a particular physician). If the machine module is trained to predict the satisfaction level for a particular physician, on the other hand, the satisfaction level may be representative of a subjective image quality specifically taking into account the preferences of the particular physician.

The result of the image quality level assessment may be displayed by the assessment component to a user (e.g., an on-site technician) with an indication of the predicted satisfaction level, so that the user may know whether the physician in charge of the examination will prospectively be satisfied with the acquired medical image or not. When the physician will likely be satisfied (e.g., when the determined level of image quality exceeds a predetermined threshold), the acquired medical image may be provided to the physician for review. This may comprise transmitting the acquired medical image to a workstation of the physician (in particular, when the physician works from a remote location). On the other hand, when the physician will likely not be satisfied (e.g., when the determined level of image quality does not exceed the predetermined threshold), the acquisition of the medical image may be re-run with an adjusted configuration of the medical imaging device within the same examination, i.e., without the patient having to leave the scanner. When acquiring the medical image is performed as part of an examination, the method may thus further comprise repeating acquiring the medical image by the medical imaging device with a different configuration within the (same) examination.

Upon receipt of the acquired medical image for review, the physician may rate the medical image using a feedback component provided in the medical imaging system to provide feedback on the quality level of the medical image as perceived by the physician. Such feedback may systematically be collected and incorporated into the collection of feedback of physicians for previously acquired medical images mentioned above. The newly collected feedback may be used to reassess or refine the selection of the plurality of reference images and/or to refine the accuracy of the machine learning module which predicts the satisfaction levels. In one variant, the method may thus further comprise collecting feedback on the image quality level of the acquired medical image from a physician and using the collected feedback for selecting reference images representative of desired image quality levels for particular types of medical images. In another variant, the method may further comprise collecting feedback on the image quality level of the acquired medical image from a physician and using the collected feedback for training the machine learning module to predict satisfaction levels of physicians or groups of physicians for medical images, as described above. A feedback loop may therefore be introduced which allows a continuous quality improvement of images acquired in the medical imaging system, even across multiple sites.

According to a second aspect, a system for configuring a medical imaging device by a set of acquisition parameters for acquiring a medical image is provided. The system comprises a composition component adapted to obtain a selection of a reference image from a plurality of reference images, each of the plurality of reference images being stored in association with a set of acquisition parameters which has been used to acquire the respective reference image, and a configuration component adapted to configure the medical imaging device by the set of acquisition parameters stored in association with the selected reference image for acquiring the medical image.

The system according to the second aspect may correspond to the medical imaging system described above in relation to the method of the first aspect. Therefore, all features described above in relation to the medical imaging system may be comprised by the system of the second aspect as well, including all aspects of the plurality of medical imaging devices, the central repository, the local repository instances and all components mentioned above, including the configuration component, the composition component, the maintenance component, the assessment component and the feedback component. Unnecessary repetitions are thus omitted in the following.

### Brief Description of the Drawings

In the following, the present disclosure will further be described with reference to exemplary embodiments illustrated in the figures, in which:
- Figure 1: illustrates an overview of a method which may be performed in a medical imaging system according to the present disclosure;
- Figure 2: illustrates an exemplary composition of a computing unit on which one or more of the components of a medical imaging system according to the present disclosure may be executed;
- Figure 3: illustrates an overview of the feedback loop in the acquisition process according to the present disclosure;
- Figure 4: illustrates an exemplary repository structure for use in a medical imaging system according to the present disclosure;
- Figure 5: illustrates an overview of a medical imaging system and its components according to the present disclosure; and
- Figure 6: illustrates an exemplary view indicating a predicted satisfaction level of a physician according to the present disclosure.

### Detailed Description

In the following description, for purposes of explanation and not limitation, specific details are set forth in order to provide a thorough understanding of the present disclosure. It will be apparent to one skilled in the art that the present disclosure may be practiced in other implementations that depart from these specific details. In particular, while the following description particularly relates to a system comprising radiological centers, it will be understood that the present disclosure is not limited thereto and that the technique presented herein may be practiced with any other medical imaging system.

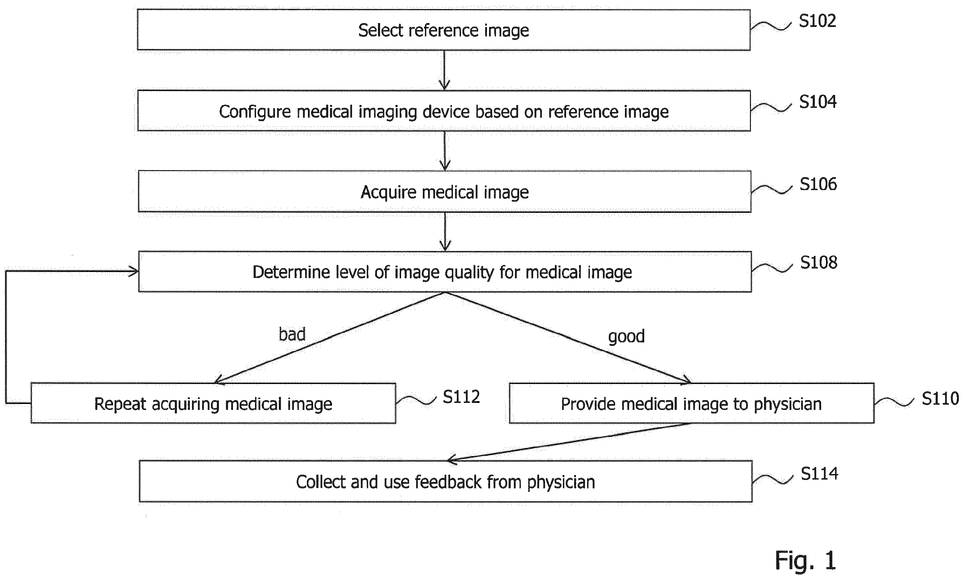

Figure 1 illustrates an overview of a method which may be performed in a medical imaging system according to the present disclosure. The method may be dedicated to configuring a medical imaging device by a set of acquisition parameters for acquiring a medical image and to performing a feedback-based quality assessment for the acquired medical image. The method of Figure 1 may generally correspond to the method described above. Unnecessary repetitions are thus omitted in the following.

In step S102, the method may comprise selecting a reference image from a plurality of reference images, wherein each of the plurality of reference images is stored in association with a set of acquisition parameters which has been used to acquire the respective reference image. In step S104, the method may comprise configuring the medical imaging device by the set of acquisition parameters stored in association with the selected reference image for acquiring the medical image. In step S106, the method may comprise acquiring the medical image by the medical imaging device. In step S108, the method may comprise determining a level of image quality of the acquired medical image using an image quality assessment component configured to assess image quality levels based on feedback of physicians collected for previously acquired medical images. When the determined level of image quality exceeds a predetermined threshold (i.e., is determined to be "good"), the method may further comprise providing, in step S110, the acquired medical image to a physician. When the determined level of image quality does not exceed the predetermined threshold (i.e., is determined to be "bad"), the method may comprise repeating, in step S112, acquiring the medical image by the medical imaging device with a different configuration within the same examination. Re-acquiring the medical image in accordance with step S112 may be performed repeatedly until the determined level of image quality, as determined in step S108, is good enough to provide the acquired medical image to the physician in accordance with step S110. In step S114, the method may further comprise collecting feedback on the image quality level of the acquired medical image from the physician and using the feedback for improvements of the medical imaging system, such as using the collected feedback for selecting reference images representative of the desired image quality levels for particular types of medical images and/or using the collected feedback for training a machine learning module to predict satisfaction levels of physicians or groups of physicians for medical images.

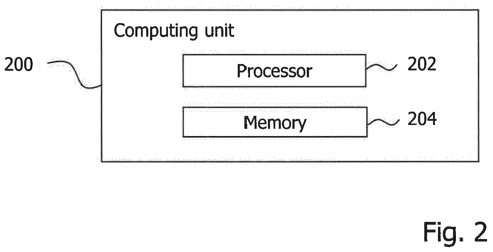

Figure 2 illustrates an exemplary composition of a computing unit 200 on which one or more of the components of the medical imaging system according to the present disclosure may be executed. Thus, each of the components of the medical imaging system described herein may be executed on a computing unit like computing unit 200. These components may in particular include the plurality of medical imaging devices, the central repository, the local repositories, the configuration component, the composition component, the maintenance component, the assessment component, and the feedback component. The computing unit 200 may comprise at least one processor 202 and at least one memory 204, wherein the at least one memory 204 may contain instructions executable by the at least one processor 202 such that the respective computing unit 200 is operable to carry out the functions of the respective component described herein.

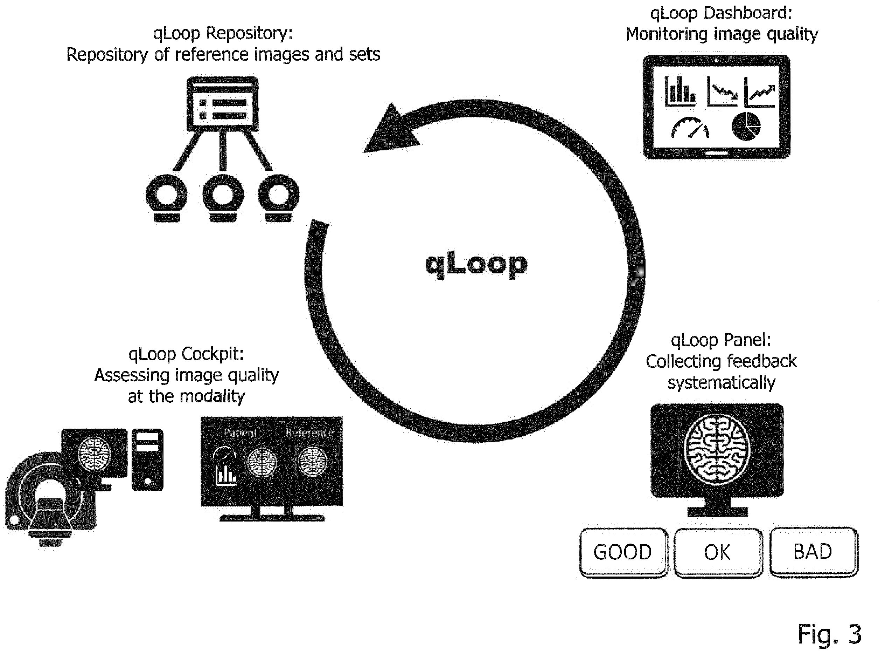

Figure 3 illustrates a general overview of the feedback loop in the acquisition process introduced by the present disclosure. The feedback loop (denoted as quality loop "qLoop" in the figure) may generally aim at measuring, controlling, assuring and/or improving the quality of medical images acquired in the medical imaging system according to the present disclosure. Acquisition parameters may be managed based on reference images instead of conventional generic acquisition protocols stored locally on the medical imaging devices. Images of "good" quality may be exported from a PACS and imported as reference images into a qLoop repository (corresponding to the "central repository" described above). Example images selected from the PACS may become templates for future image acquisitions and the acquisition process may thus generally be denoted as "scan by example". Configuring the medical imaging device by reference images may allow radiologists to define the desired acquisition parameters in an easy and intuitive manner, i.e., by just pointing to a representative sample image which has been rated to be "good". Instead of configuring a generic acquisition protocol stored locally at the medical imaging device, the radiologist may thus not need to be physically at the imaging device but may rather select the reference image for configuring the medical imaging device from a remote location.

Besides mere reference images, the repository may contain predefined collections of reference images, called "sets" (corresponding to the "sequences of reference images" described above). Such sets may combine reference images into a program, i.e., into a sequence of instructions for the imaging device to acquire a series of medical images required for a complete examination of a patient. The repository including the reference images and sets may be shared and synchronized between multiple radiological centers so that radiologists may benefit from the above-mentioned advantages in all radiological centers of the system.

For a specific examination, a reference image or set may be selected (and, optionally, adjusted, such as by adapting acquisition parameters associated with a reference image or by adding/removing reference images from a set) by the radiologist using the composition component. The reference image or the set may then be sent to the imaging device to configure the imaging device accordingly and start the acquisition. After the acquisition, the acquired image or sequence of images may be quality controlled at a qLoop cockpit (corresponding to the "assessment component" described above) by applying automatic image quality checks based on feedback of physicians collected for previously acquired medical images (and, optionally, based on conventional quantitative image quality measurements).

If the acquired image or sequence of images passes the quality check, it may be sent for review to the radiologist who may evaluate its quality as part of the usual reading and who may rate the quality at a qLoop panel (corresponding to the "feedback component" described above) by indicating a corresponding level of satisfaction (cf. "good", "ok" and "bad" in Figure 3). Such feedback of radiologists may be systematically collected, centrally monitored at a qLoop dashboard and used to identify improvement potential with respect to the selection of reference images in the repository. In the centrally-managed repository, changes reflecting such improvements may be introduced to thereby close the qLoop and provide for a continuous process of improving reference images based on feedback given by radiologists. The feedback process may generally be scalable, i.e., an unlimited number of radiologists may provide feedback in parallel for images acquired in multiple radiological centers.

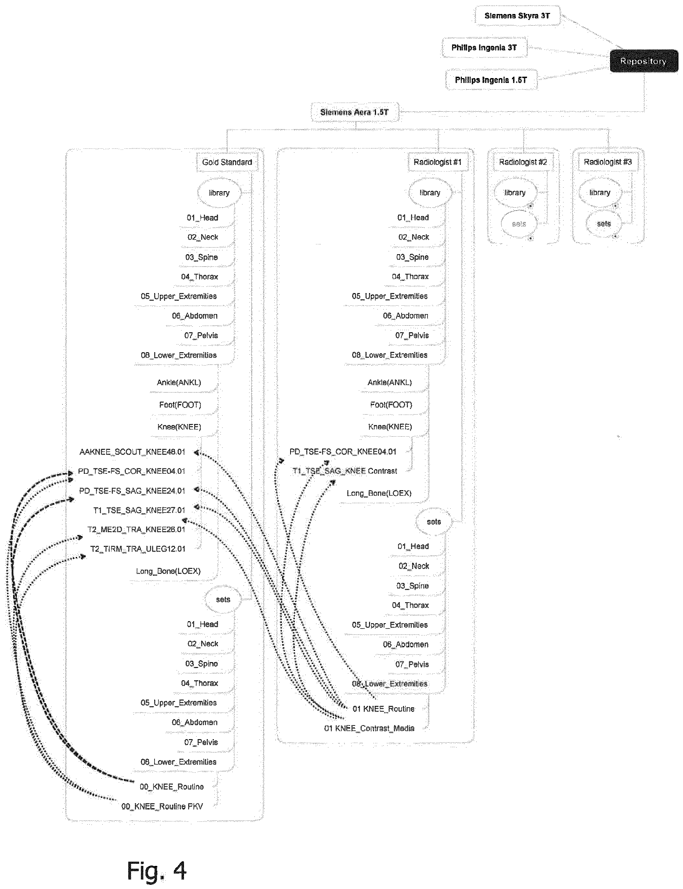

An exemplary structure of the repository is depicted in Figure 4 which shows that the repository may be organized in accordance with plural categories. In the shown example, the categories are reflected by "levels" to provide support for different imaging devices types and to facilitate the retrieval of reference images. In the example of Figure 4, the repository supports an indication of medical imaging device types at the top level (corresponding to the category "medical imaging device type" described above), like Siemens Area 1.5T, Philips Ingenia 3T, etc. At the second level, the repository supports an indication of a standard image set representative of generally recommended acquisition parameters (cf. "gold standard" in the figure, corresponding to the category "standard image set" described above) as well as indications of physician specific image sets representative of acquisition parameters preferred by a particular physician (corresponding to the category "physician specific image set" described above). Due to the feedback loop, the reference images of the standard image set may be improved over time and, if a particular radiologist has specific needs, specific reference images may be stored under the physician specific image set of that radiologist. At the third level of the repository, the actual reference images may be stored (cf. the "library" sections in the figure). At this level, the reference images may be organized with respect to body regions, e.g., head, lower extremities, etc., (corresponding to the category "body region" described above) as well as actual anatomical parts to be scanned, e.g., ankle, foot, knee, etc. (corresponding to the category "anatomical part" described above). Each reference image may be stored in association with an SOP which contains instructions regarding the required patient positioning, or the like. Sequences of reference images, i.e., sets, representing a series of acquisitions may be stored in the "sets" section at the third level. Similar to the reference images in the "library" section, the "sets" section may be organized with respect to body regions and anatomical parts and each set may be stored in association with a corresponding SOP.

The arrows in Figure 4 visualize exemplary links from sets of the "sets" section to reference images in the "library" section, illustrating that reference images of the sets may be stored by reference in order to avoid duplicate storage of reference images. For example, the set "00_KNEE_Routine" includes a link to the reference images "PD_TSE-FS_COR_KNEE04.01" and "PD_TSE-FS_SAG_KNEE24.01" included in the "library" section of the standard image set. Links may also range from a radiologist specific set (cf. "01 KNEE_Contrast_Media" in the figure) to reference images in the library of the standard image set, for example.

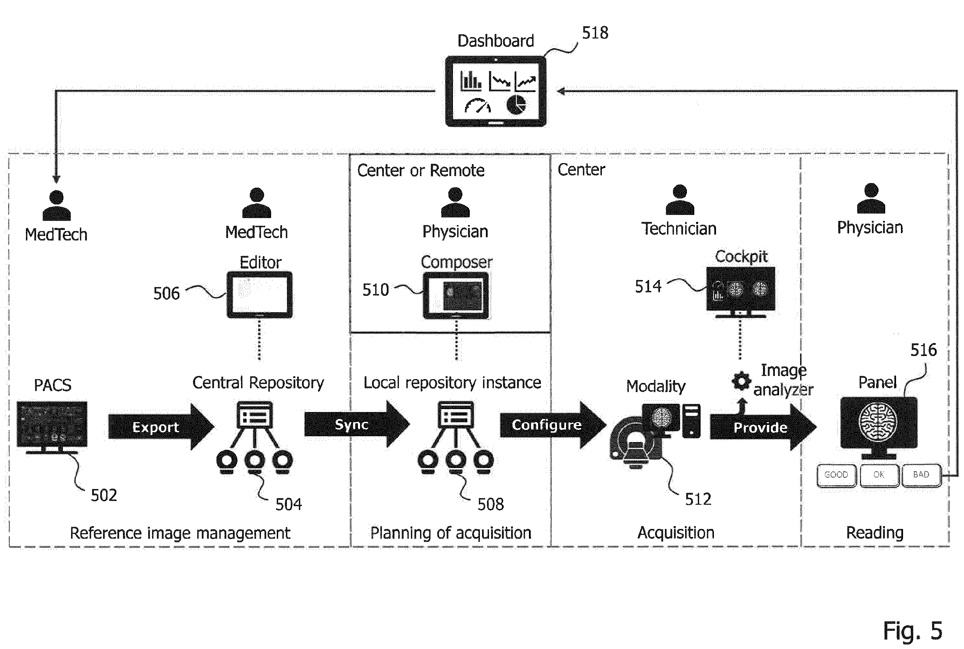

Figure 5 illustrates an overview of a medical imaging system according to the present disclosure. In the medical imaging system, a PACS 502 may serve as a source for selecting reference images. From the PACS 502, medical images of "good" quality may be exported together with their associated acquisition parameters and incorporated as reference images into the central repository 504. Using an editor component 506 (corresponding to the "maintenance component" described above), the consistency of the repository may be maintained, e.g., links to reference images may be kept up-to-date and reference images with similar acquisition parameters may be identified. If reference images with sufficiently similar acquisition parameters are found, such reference images may be removed from the repository and corresponding links may be updated to prevent a degenerated repository.

Each radiological center of the system may maintain a local repository instance 508 which may be synchronized with the central repository 504, i.e., each diagnostic center may store a local subset of the central repository 504, e.g., depending on which radiologists work in this center. The local repository instance 508 may form the basis to select a reference image or a set for a specific examination using a composer component 510 (corresponding to the "composition component" described above). The composer component 510 may include a user interface for use by the radiologist assisting in performing corresponding selections. If required, the composer component 510 may be usable to adjust acquisition parameters of the selected reference image or to adjust the sequence of reference images of a set (e.g., by adding/removing reference images from the set) to thereby create a patient specific examination. The composer component 510 may be operated by the radiologist at the radiological center or from remote.

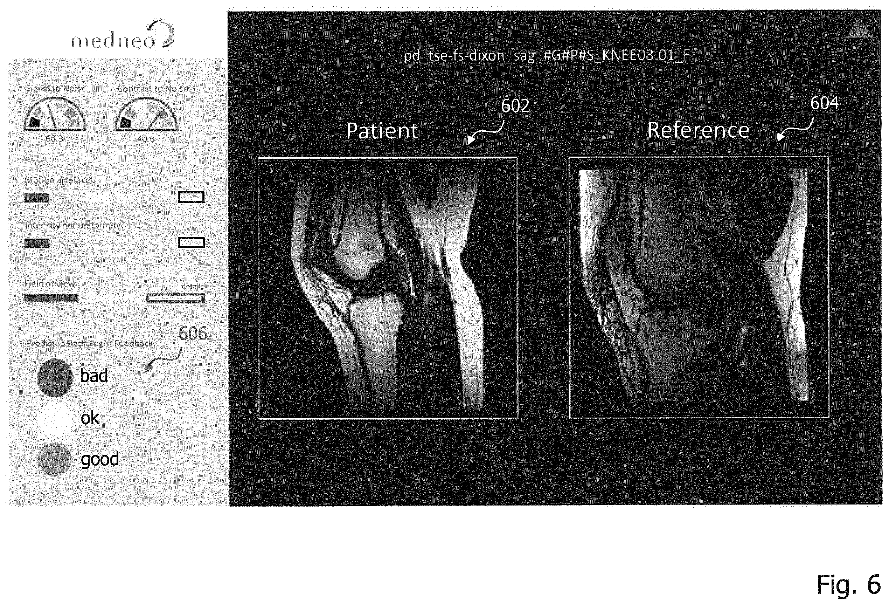

The selected reference image or set may then be sent to the medical imaging device 512, e.g., via a DICOM-based transfer, and the medical imaging device 512 may automatically be configured based on the selected reference image or set. The reference image or set may also be displayed in a DICOM browser at the medical imaging device 512 together with the corresponding SOP. The technician at the medical imaging device 512 may thus prepare the acquisition in accordance with the SOP and perform the actual acquisition process as usual. At the end of the acquisition process, the medical imaging device 512 may send the acquired image or images to the cockpit component 514 (corresponding to the "assessment component" described above) which may determine a corresponding image quality level. The cockpit component 514 may provide a user interface to the technician to display the result of the image quality level assessment, e.g., including an indication of the predicted satisfaction level of the radiologist in charge, so that the technician may know whether or not the radiologist will prospectively be satisfied with the acquired medical image or images. Such display is exemplarily illustrated in Figure 6, where the acquired medical image 602 is displayed next to the corresponding reference image 604 for visual comparison by the technician, and where the predicted satisfaction level of the radiologist is displayed in a traffic light-like manner in the lower left portion of the figure (cf. reference numeral 606). In the upper left portion of the figure, additional quality measurement results are displayed, which may be calculated based on conventional quantitative quality measurements, for example. In the example shown, these measurements comprise assessments of the signal-to-noise ratio, contrast-to-noise ratio, motion artifacts, intensity of nonuniformity, and field of view.

Based on these results, in particular based on the predicted satisfaction level of the radiologist, the technician may decide on whether the acquisition needs to be repeated or whether the image or images may be sent to the radiologist for review. As described above, the predicted satisfaction level of the radiologist may be determined using artificial intelligence-based techniques like deep learning and vector machines, for example. In particular, corresponding algorithms may be trained to predict the satisfaction of a group of radiologists or a single radiologist after an adequate amount of feedback has been collected. The acquired image or images may be sent to the radiologist who may read the image as usual. During the reading process, the radiologist may rate the image quality based on his own assessment on a panel component 516 (corresponding to the "feedback component" described above). The panel component 516 may provide corresponding input means for the radiologist to submit this feedback. The feedback may then be stored (e.g., in the PACS 502 or another dedicated storage), monitored at a dashboard component 518, and used to improve the identification of images with good acquisition parameters for the selection of future reference images as well as to optimize future satisfaction level predictions of radiologists. The feedback given by the radiologists may thus provide a basis for continuous improvements of the reference images and, hence, of the quality level of images acquired in the medical imaging system in general.

As has become apparent from the above, the present disclosure provides a technique for configuring a medical imaging device by a set of acquisition parameters for acquiring a medical image and for performing a feedback-based quality assessment of the acquired medical image. According to the presented technique, acquisition parameters may be defined based on reference images rather than based in the conventional generic acquisition protocols locally stored on the modalities. On-site technicians may thus not need to choose a generic acquisition protocol from a locally installed library and mistakes, such as applying wrong acquisition parameters, may thus generally be avoided. By synchronizing reference images from a centrally managed repository across different radiological centers, it may further be ensured that the same acquisition parameters are applied across all sites and that obsolete acquisition parameters are consistently removed from all sites. Patient specific adjustments may be defined by radiologists at their desks and may not have to be applied by the on-site technicians so that inappropriate adjustments of acquisition parameters may eliminated. Besides the reference images, corresponding SOPs associated with the acquisition parameters may be stored in the central repository and presented to the technician prior to image acquisition. Applied SOPs may thus always be up-to-date and assist the technicians in properly performing tasks, such as positioning the patient in the scanner or preparing the acquisition accordingly.

By the technique presented herein, the technician may be presented with predicted satisfaction levels of the radiologists in charge for the acquired images. The predicted satisfaction levels may not only be assessed quantitatively but may be based on feedback of radiologists collected for previously acquired medical images, which may reflect the required image quality more precisely and which may even reflect subjective preferences of the radiologists, if desired. Technicians may additionally be notified about image artifacts, wrong fields of view, or other image quality issues and may thus decide on whether the acquisition should be re-run before the images are sent to the radiologist and as long as the patient is still in the scanner. The feedback provided by radiologists may systematically be collected, monitored and incorporated into the selection process of reference images and used to refine the prediction for the satisfaction levels of radiologists. In this way, improvement potential for reference images may be detected in a continuous feedback loop and contribute to improving the overall image quality available in the system.

It is believed that the advantages of the technique presented herein will be fully understood from the foregoing description, and it will be apparent that various changes may be made in the form, constructions and arrangement of the exemplary aspects thereof without departing from the scope of the disclosure or without sacrificing all of its advantageous effects. Because the technique presented herein can be varied in many ways, it will be recognized that the disclosure should be limited only by the scope of the claims that follow.

## Claims

1. A method for configuring a medical imaging device (512) by a set of acquisition parameters for acquiring a medical image (602), the method comprising:
selecting (S102) a reference image (604) from a plurality of reference images (604), each of the plurality of reference images (604) being stored in association with a set of acquisition parameters which has been used to acquire the respective reference image (604); and
configuring (S104) the medical imaging device (512) by the set of acquisition parameters stored in association with the selected reference image (604) for acquiring the medical image (602).

2. The method of claim 1, wherein each of the plurality of reference images (604) is previously selected as being representative of a desired level of image quality for a particular type of medical image.

3. The method of claim 1 or 2, wherein the medical imaging device (512) is one of a plurality of medical imaging devices (512) distributed across a plurality of sites and wherein the plurality of reference images (604) is stored in a central repository (504) configured to distribute the plurality of reference images (604) to the plurality of medical imaging devices (512).

4. The method of claim 3, wherein, in each of the plurality of sites, a local subset of the central repository (504) is maintained, wherein each local subset is synchronized with the central repository (504).

5. The method of claim 3 or 4, wherein, in the central repository (504), the plurality of reference images (604) is categorized by at least one of:
medical imaging device types,
standard image sets and/or physician specific image sets,
body regions, and
anatomical parts.

6. The method of any one of claims 1 to 5, wherein each of the plurality of reference images (604) is stored in association with a standard operating procedure, SOP, to be adhered to when acquiring the medical image (602) using the set of acquisition parameters stored in association with the respective reference image (604).

7. The method of any one of claims 3 to 6, wherein, in the central repository (504), sequences of reference images (604) are defined, wherein the medical imaging device (512) is configurable by each of the sequences to acquire a respective series of medical images (602) in accordance with the sets of acquisition parameters stored in association with the reference images (604) of the respective sequence.

8. The method of claim 7, wherein reference images (604) of the sequences are stored by reference to corresponding reference images (604) of the plurality of reference images (604).

9. The method of any one of claims 1 to 8, further comprising:
acquiring (S106) the medical image (602) by the medical imaging device (512); and
determining (S108) a level of image quality for the acquired medical image (602) using an assessment component (514) configured to assess image quality levels based on feedback of physicians collected for previously acquired medical images.

10. The method of claim 9, wherein the assessment component (514) comprises a machine learning module trained to predict a satisfaction level of a physician or a group of physicians based on the feedback of physicians collected for previously acquired medical images.

11. The method of claim 9 or 10, further comprising:
providing (S110) the acquired medical image (602) to a physician when the determined level of image quality exceeds a predetermined threshold.

12. The method of any one of claim 9 to 11, wherein acquiring (S106) the medical image (602) is performed as part of an examination and wherein the method further comprises:
repeating (S112) acquiring the medical image (602) by the medical imaging device (512) with a different configuration within the examination when the determined level of image quality does not exceed a predetermined threshold.

13. The method of any one of claims 9 to 12, further comprising:
collecting (S114) feedback on the image quality level of the acquired medical image (602) from a physician and using the collected feedback for selecting reference images (604) representative of desired image quality levels for particular types of medical images.

14. The method of any one of claims 10 to 13, further comprising:
collecting (S114) feedback on the image quality level of the acquired medical image (602) from a physician and using the collected feedback for training the machine learning module to predict satisfaction levels of physicians or groups of physicians for medical images.

15. A system for configuring a medical imaging device (512) by a set of acquisition parameters for acquiring a medical image (602), the system comprising:
a composition component adapted to obtain a selection of a reference image (604) from a plurality of reference images (604), each of the plurality of reference images (604) being stored in association with a set of acquisition parameters which has been used to acquire the respective reference image (604); and
a configuration component adapted to configure the medical imaging device (512) by the set of acquisition parameters stored in association with the selected reference image (604) for acquiring the medical image (602).
